(19)

Europäisches
Patentamt
European
Patent Office
Office européen
des brevets

(11)  **EP 2 762 057 A1**

(12)  # EUROPEAN PATENT APPLICATION

(43) Date of publication:
**06.08.2014 Bulletin 2014/32**

(21) Application number: **14152982.6**

(22) Date of filing: **29.01.2014**

(51) Int Cl.:
*A61B 1/00* (2006.01)      *G02B 23/24* (2006.01)
*H04N 13/00* (2006.01)      *H04N 13/02* (2006.01)

| | |
|---|---|
| (84) Designated Contracting States:<br>**AL AT BE BG CH CY CZ DE DK EE ES FI FR GB<br>GR HR HU IE IS IT LI LT LU LV MC MK MT NL NO<br>PL PT RO RS SE SI SK SM TR**<br>Designated Extension States:<br>**BA ME**<br><br>(30) Priority: **04.02.2013 JP 2013019424** | (71) Applicant: **Canon Kabushiki Kaisha**<br>**Tokyo 146-8501 (JP)**<br><br>(72) Inventor: **Katayama, Akihiro**<br>**Tokyo 146-8501 (JP)**<br><br>(74) Representative: **TBK**<br>**Bavariaring 4-6**<br>**80336 München (DE)** |

(54)  **Stereo endoscope apparatus and image processing method**

(57)      Provided is a stereo endoscope apparatus, including: a treatment instrument that is operable; at least two imaging units for picking up images of a subject; and a transparency-composition unit for performing, in a first region in which the treatment instrument appears within a first image picked up by one of the at least two imaging units, image composition by blending, at a predetermined composition ratio, an image of the first region and an image of a second region in which the treatment instrument does not appear within a second image picked up by another of the at least two imaging units.

# FIG. 1

EP 2 762 057 A1

**Description**

BACKGROUND OF THE INVENTION

Field of the Invention

The present invention relates to a stereo endoscope apparatus and an image processing method.

Description of the Related Art

A related-art stereo endoscope includes a stereo camera, a channel for inserting a treatment instrument, and a lighting device at a tip of the endoscope to be inserted into a subject to be observed. A doctor performs surgery using the treatment instrument and the like while observing a stereo image on a monitor.

In such stereo endoscope, for an object that is brought into close proximity with the stereo camera, such as the treatment instrument, a convergence angle determined by the stereo camera becomes too large, which makes stereoscopy difficult. To address this problem, there have hitherto been known a method involving forming a two-dimensional image at only a predetermined area and a method involving generating and superimposing a mask image. Further, Japanese Patent Application Laid-Open No. 2012-223446 discloses that right and left images are replaced with each other to delete a problematic region of the treatment instrument, or that an image to be displayed is transformed to perform adjustment so that the parallax is reduced.

However, the hitherto known method involves determining a difficult region for the stereoscopy in advance and displaying only a monocular image for the region, or superimposing the mask image on the region. Therefore, there is a problem in that, even though only a small part of the treatment instrument is delivered through the channel, the region other than the treatment instrument is also displayed as a two-dimensional image or masked. Further, in the method disclosed in Japanese Patent Application Laid-Open No. 2012-223446, control is performed so that the tip portion of the treatment instrument is constantly visible, and hence, when this tip portion is located in the region difficult for the stereoscopy, the feeling of visual field interference may become stronger.

SUMMARY OF THE INVENTION

The present invention has been made in view of the above-mentioned problems. That is, the present invention may provide a stereo endoscope apparatus configured to perform, in a region causing a strong feeling of visual field interference, transparency-composition processing using an image in which a treatment instrument appears and an image in which the treatment instrument does not appear, thereby being capable of alleviating the feeling of visual field interference while an observer is recognizing the treatment instrument, and an image processing method thereof.

Therefore, according to a first aspect of the present invention, there is provided a stereo endoscope apparatus, including: a treatment instrument that is operable; at least two imaging units for picking up images of a subject; and a transparency-composition unit for performing, in a first region in which the treatment instrument appears within a first image picked up by one of the at least two imaging units, image composition by blending, at a predetermined composition ratio, an image of the first region and an image of a second region in which the treatment instrument does not appear within a second image picked up by another of the at least two imaging units.

Further, according to a second aspect of the present invention, there is provided an image processing method for a stereo endoscope apparatus including a treatment instrument that is operable, the image processing method including: picking up images of a subject by at least two imaging units; and performing, in a first region in which the treatment instrument appears within a first image picked up by one of the at least two imaging units, image composition by blending, at a predetermined composition ratio, an image of the first region and an image of a second region in which the treatment instrument does not appear within a second image picked up by another of the at least two imaging units.

Further features of the present invention will become apparent from the following description of exemplary embodiments with reference to the attached drawings.

BRIEF DESCRIPTION OF THE DRAWINGS

FIG. 1 is a functional block diagram of a processing system according to a first embodiment of the present invention.

FIG. 2 is an enlarged view of an example of a stereo endoscope (only a tip portion thereof) according to the first embodiment.

FIGS. 3A, 3B, and 3C are schematic diagrams illustrating processing to be performed by a transparency-composition unit in the processing system according to the first embodiment.

FIG. 4 is a flowchart of processing according to the first embodiment.

FIG. 5 is a functional block diagram of a processing system according to a second embodiment of the present invention.

FIGS. 6A, 6B, and 6C are explanatory diagrams illustrating processing to be performed by a tip-area detecting unit in the processing system according to the second embodiment.

FIGS. 7A and 7B are explanatory diagrams illustrating the processing to be performed by the tip-area detecting unit in the processing system according to the second embodiment.

FIG. 8 is a flowchart of processing according to the second embodiment.

FIG. 9 is a functional block diagram of a processing system according to a sixth embodiment of the present invention.

FIGS. 10A, 10B, 10C, and 10D are schematic diagrams illustrating an example of image transformation processing according to the sixth embodiment.

FIG. 11 is a flowchart of processing according to the sixth embodiment.

FIGS. 12A, 12B, 12C, and 12D are schematic diagrams illustrating another example of the image transformation processing according to the sixth embodiment.

FIG. 13 is a functional block diagram of a processing system according to a seventh embodiment of the present invention.

FIG. 14 is a diagram illustrating an example of a geometric model of a treatment instrument.

FIG. 15 is a flowchart of processing according to the seventh embodiment.

FIG. 16 is a functional block diagram of a processing system according to an eighth embodiment of the present invention.

FIGS. 17A, 17B, and 17C are schematic diagrams illustrating treatment instrument deletion processing according to the eighth embodiment.

FIG. 18 is a flowchart of processing according to the eighth embodiment.

FIGS. 19A and 19B are explanatory diagrams illustrating treatment instrument deletion processing according to a ninth embodiment of the present invention.

FIG. 20 is a block diagram of a computer to be used in another embodiment of the present invention.

DESCRIPTION OF THE EMBODIMENTS

Now, referring to the attached drawings, a stereo endoscope apparatus and an image processing method according to exemplary embodiments of the present invention are described in detail. However, the scope of the present invention is not limited to the examples illustrated in the drawings.

First embodiment

A stereo endoscope apparatus according to a first embodiment of the present invention is configured to perform, in a region causing a strong feeling of visual field interference, transparency composition using an image in which a treatment instrument appears and an image in which the treatment instrument does not appear. The transparency composition herein refers to image composition that involves blending the images at a predetermined composition ratio (blend ratio). An image H generated by performing the transparency composition using an image F and an image G can be expressed by the following equation (the image G is presumed as an image having the same pixel size as the image F). In the equation, "F(i,j)" represents a pixel value of the image F, "G(i,j)" represents a pixel value of the image G, "H(i,j)" represents a pixel value of the image H, and "transparency" represents a transparency ratio. Further, "i" and "j" are integer numbers satisfying 0<i<width+1 and 0<j<height+1, "width" representing the number of pixels in a width direction in the image F and "height" representing the number of pixels in a height direction in the image F.

H(i, j) = (1.0 transparency * F(i, j) - transparency * C(i, j)

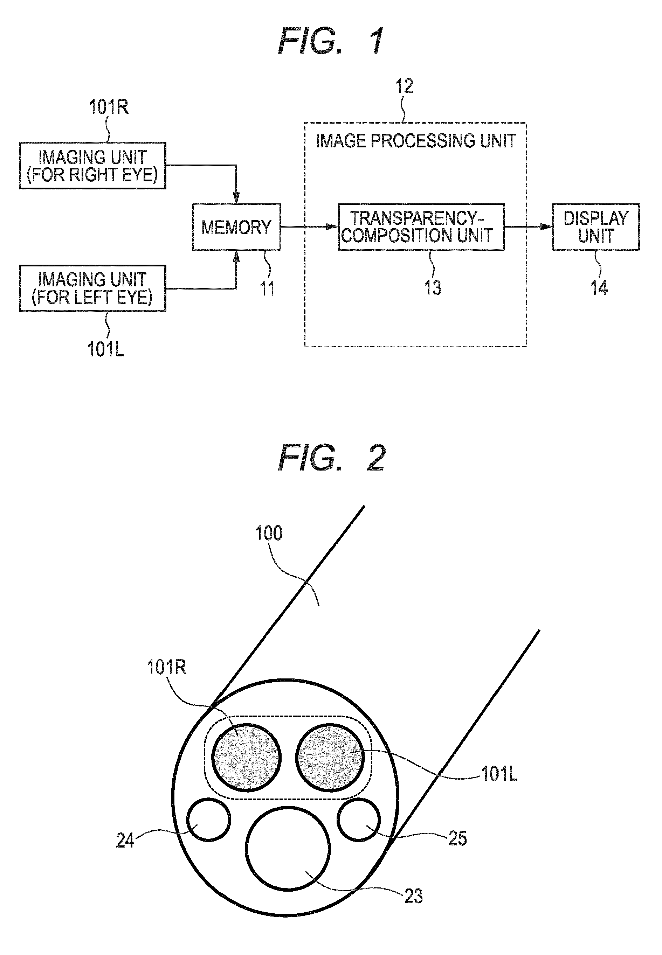

FIG. 1 is a functional block diagram of a processing system according to this embodiment. FIG. 1 illustrates imaging units (cameras) 101R and 101L of an endoscope for picking up images of a subject, and a memory 11. Further, FIG. 1 illustrates an image processing unit 12 for processing the picked-up images. The image processing unit 12 includes a transparency-composition unit 13 for performing transparency composition using the image in which the treatment instrument appears and the image in which the treatment instrument does not appear. Further, FIG. 1 illustrates a stereo image display unit 14 for displaying an image obtained through the transparency composition. The image processing unit 12 in this case may be realized through use of, for example, a CPU. With this configuration, the respective images captured by the at least two imaging units 101R and 101L are temporarily stored in the memory 11. The images stored in the memory 11 are sent to the transparency-composition unit 13. In the region causing a strong feeling of visual field interference within the stored image, the transparency-composition unit 13 performs the transparency composition using images of that region and a region having the highest correlation with that region within the image picked up by the other imaging unit. The display unit 14 displays an image obtained through the transparency composition in three dimensions. Note that, an existing stereo image display device may be used as the display unit 14.

FIG. 2 illustrates a tip portion 100 of the stereo endoscope. The camera 101R for a right eye, the camera 101L for a left eye, a channel 23 that allows an operable treatment instrument to pass therethrough, and lighting devices 24 and 25 are provided at the tip portion 100 of the endoscope.

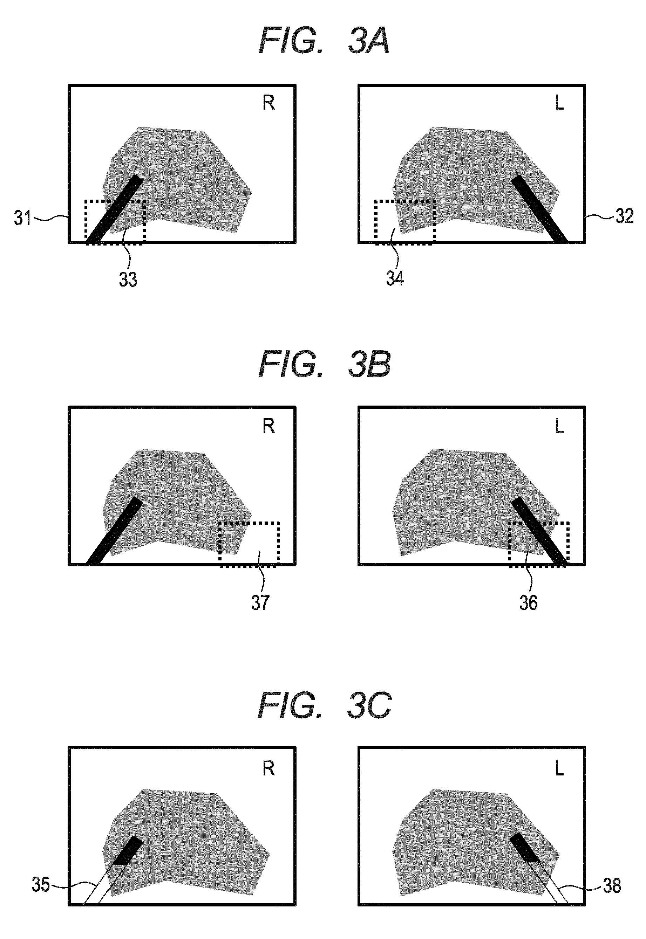

Referring to FIGS. 3A to 3C, processing to be performed by the transparency-composition unit 13 is described. FIG. 3A illustrates a region (a first region) 33 causing a strong feeling of visual field interference within an image 31 (a first image) for the right eye, and a region (a second region) 34 having the highest correlation with the region 33 within an image 32 (a second image) for the left eye. Referring to FIG. 3A, it is understood that the treatment instrument does not appear in the region 34 having the highest correlation with the region 33. The positional relationship between the camera of the imaging unit and the channel is fixed, and hence a track of the treatment instrument to be delivered is substantially constant. Thus, the region 33 causing a strong feeling of visual field interference may be determined in advance.

Subsequently, the transparency-composition unit 13 generates an image that a region in which the treatment instrument appears within the region 33 is masked, and calculates correlation between the image obtained through the masking and the image 32 for the left eye. Note that, the masked region of the treatment instrument is excluded in the calculation of correlation. As a method of calculating of correlation between the image obtained through the masking and the image 32 for the left eye, there may be used a method of performing region-based matching within the image 32 for the left eye through use of the image obtained through the masking. Note that, the sum of absolute differences (SAD), the sum of squared differences (SSD) and the like may be used for the region-based matching. Further, as illustrated in FIG. 3A, the transparency-composition unit 13 identifies the region 34 having the highest correlation. The region 34 having high correlation may be identified every time the images are picked up. Alternatively, the position of the previously identified region may be stored, and the stored position of the previously identified region may be used in common to identify the region 34 in the subsequent processing.

After the region 34 having high correlation is identified, in the region 33 within the image 31 for the right eye, the transparency-composition unit 13 performs the transparency composition using the image of the region 33 and the image of the region 34. The transparency composition is achieved by combining the pixel values of the image of the region 33 and the pixel values of the image of the region 34 at a predetermined composition ratio. In this case, the pixel values are combined at a composition ratio of, for example, 1:9. Thus, as illustrated in FIG. 3C, the transparency-composition unit 13 may generate such an image that an region in which the treatment instrument appears within the region 33 becomes transparent as in a region 35. As the degree of how transparent the region is, "transparency" is defined. The transparency is represented by a real number within a range of "0≤transparency≤1", where "0" indicates complete opacity, and "1" indicates complete transparency. In the example described above, the transparency of the image of the region 33 that is subjected to the transparency composition is 0.9. However, the composition ratio of the respective pixels is not limited thereto, and may be set arbitrarily. For example, even when the transparency of the image of the region 33 is 0.4, since the image of the region 33 is subjected to the transparency composition with the image of the region 34, the feeling of visual field interference is alleviated in the image in the region 33 after the transparency composition. Note that, the transparency of the image may be changed through the change of the composition ratio of the transparency composition.

Further, the transparency-composition unit 13 performs similar processing on the image 32 for the left eye as illustrated in FIGS. 3B and 3C. That is, the transparency-composition unit 13 determines in advance a region 36 causing a strong feeling of visual field interference within the image 32 for the left eye, and identifies a region 37 having high correlation with the region 36 within the image 31 for the right eye. After the region 37 is identified, in the region 36 within the image 32 for the left eye, the transparency-composition unit 13 performs the transparency composition using the image of the region 36 and the image of the region 37. Thus, the transparency-composition unit 13 may generate such an image that a region in which the treatment instrument appears within the region 36 causing a strong feeling of visual field interference becomes transparent in accordance with the composition ratio as in a region 38. Thus, it is possible to alleviate the feeling of visual field interference in the stereoscopy. Note that, the transparency composition in this case is performed directly using the image of the region causing a strong feeling of visual field interference and the image of the region having high correlation therewith, but the treatment instrument may be deleted from the region causing a strong feeling of visual field interference once, and then the transparency composition may be performed using the image of the region from which the treatment instrument is deleted and the image of the region in which the treatment instrument appears.

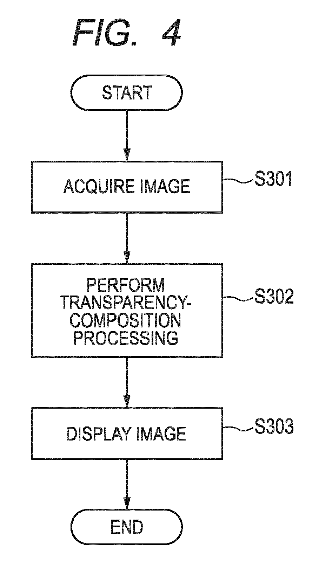

FIG. 4 is a flowchart of processing according to this embodiment.

First, in Step S301, the imaging units 101R and 101L acquire images for both eyes, and the acquired images are stored in the memory 11.

Subsequently, in Step S302, the transparency-composition unit 13 performs the transparency composition using the respective images for both eyes that are stored in the memory 11. In this case, in the region causing a strong feeling of visual field interference, the transparency-composition unit 13 performs the transparency composition through the image composition that involves blending, at a predetermined composition ratio, the image of that region and the image of the region having high correlation with that region within the image for the other eye.

After an image is generated through the transparency composition, in Step S303, the display unit displays the image generated through the transparency composition.

As described above, in the region causing a strong feeling of visual field interference, the image in which the treatment instrument appears and the image in which the treatment instrument does not appear are subjected to the transparency composition. Thus, it is possible to generate such an image that the treatment instrument becomes transparent in accordance with the composition ratio. Therefore, it is possible to alleviate the feelings of visual field interference and fatigue while the observer is recognizing the treatment instrument. Note that, when the composition ratio of the image in which the treatment instrument appears is set lower, the observer may recognize the treatment instrument with higher transparency, and hence the observer may focus on the background without stress. As a result, the feeling of visual field interference may further be alleviated.

Second embodiment

In a second embodiment of the present invention, the transparency-composition processing of the first embodiment is performed so that the tip of the treatment instrument is tracked and the range of a region in which the transparency-composition processing is performed and the composition ratio (transparency) are changed in accordance with the position of the tip.

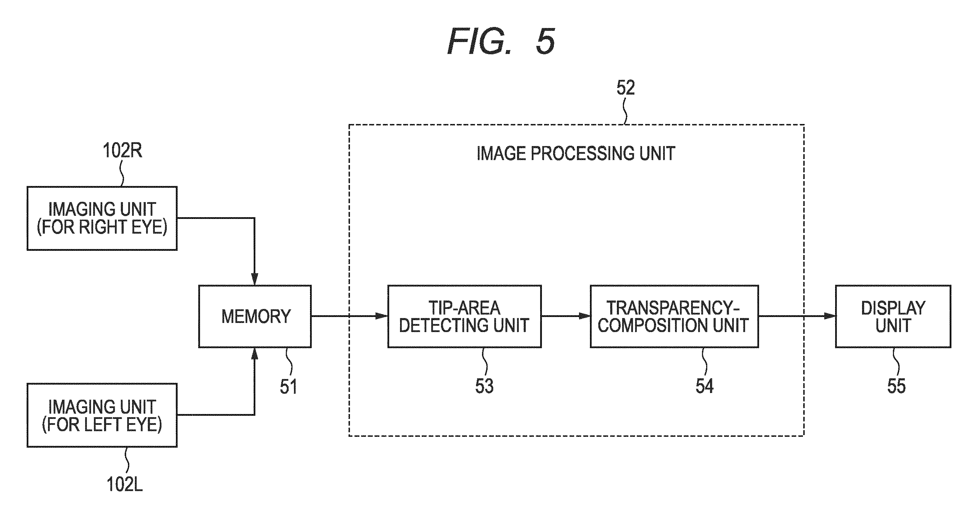

FIG. 5 is a functional block diagram of a processing system according to this embodiment. FIG. 5 illustrates an imaging unit 102R for a right eye, an imaging unit 102L for a left eye, and a memory 51. Further, FIG. 5 illustrates an image processing unit 52 for processing images. The image processing unit 52 includes a tip-area detecting unit 53 for detecting a tip area of the treatment instrument within the images picked up by the imaging units, and also includes a transparency-composition unit 54. Further, FIG. 5 illustrates a display unit 55. The image processing unit 52 in this case may be realized through use of, for example, a CPU. The imaging units 102R and 102L, the memory 51, and the display unit 55 are equivalent to those described in the first embodiment, and description thereof is therefore omitted herein.

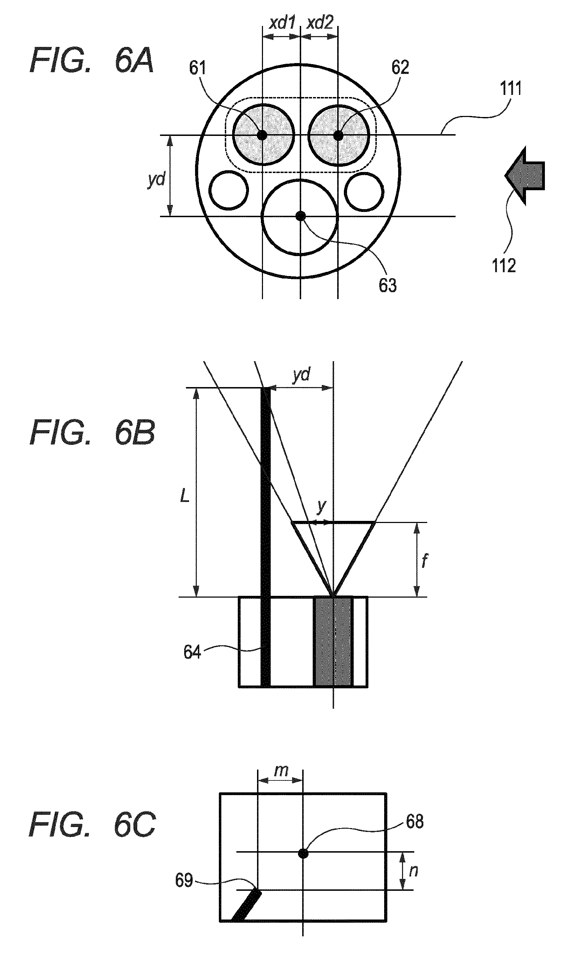

To describe operations of this embodiment, the imaging units of the stereo endoscope are first described in detail. FIG. 6A illustrates the imaging units of the stereo endoscope. FIG. 6A illustrates a center 61 of the imaging unit 102R, a center 62 of the imaging unit 102L, and a center 63 of the channel 23. The distance from the center 63 to a straight line 111 connecting the center 61 and the center 62 is represented by "yd". Further, the distance from the center 61 to the intersection between the straight line 111 and the perpendicular line extending from the center 63 to the straight line 111 is represented by "xd1", and the distance from the center 62 to the intersection between the straight line 111 and the perpendicular line extending from the center 63 to the straight line 111 is represented by "xd2". FIG. 6B is a schematic diagram illustrating a state in which a treatment instrument 64 is delivered through the channel by a length L as seen in a direction of an arrow 112 in FIG. 6A. FIG. 6C illustrates an image picked up by the imaging unit 102R. FIG. 6C illustrates a center pixel 68 of the image, and the tip 69 of the treatment instrument within the image. The values "m" and "n" represent the pixel position of the tip 69 of the treatment instrument as seen from the center pixel 68.

To simplify the description, the camera is assumed to be a pinhole camera, and the thickness of the treatment instrument is ignored. Further, it is assumed that "f" represents a distance from the camera to the image plane, "p" represents a pitch of the pixels on the imaging plane (image pickup plane), and "y" represents a position of the tip portion on the image plane (distance from the center pixel 68 on the image plane) when the treatment instrument 64 is delivered by the length L. At this time, the following relationship is established.

$$n = \frac{y}{p} = \frac{f \cdot \mathrm{yd}}{p \cdot \mathrm{L}}$$

Similarly, the following relationship is established.

$$m = \frac{f \cdot xd1}{p \cdot \mathrm{L}}$$

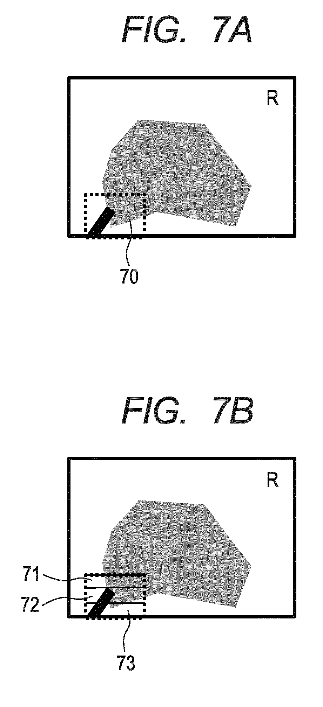

The operation of the tip-area detecting unit 53 is described. The tip-area detecting unit 53 generates a template of the image of the treatment instrument in advance, and performs template matching on a track of (m, n) expressed by Expression 1 and Expression 2 in the image stored in the memory 51, to thereby detect the position of the tip of the treatment instrument within the stored image. In this case, the image picked up by the imaging unit 102R is used alone, but the position of the tip of the treatment instrument may similarly be detected within the image picked up by the imaging unit 102L, and the detected positions may be checked by comparing with each other. As a result, it can be expected that an even higher accuracy may be achieved. Then, the tip-area detecting unit 53 sends information on the detected tip area of the treatment instrument to the transparency-composition unit 54. For example, as illustrated in FIG. 7B, the tip-area detecting unit 53 may determine a region 72 located at a predetermined distance from the tip of the treatment instrument thus detected, and a region 73 located at a distance larger than the predetermined distance described above, and send information on the regions 72 and 73 to the transparency-composition unit 54. At this time, the tip-area detecting unit 53 may also send, to the transparency-composition unit 54, information on a region 71 other than the regions 72 and 73 within a region 70 illustrated in FIG. 7A (corresponding to the region 33 causing a strong feeling of visual field interference in the first embodiment).

Next, the operation of the transparency-composition unit 54 is described. Based on the position of the tip area of the treatment instrument that is sent from the tip-area detecting unit 53, the transparency-composition unit 54 controls the composition ratio of the image composition for the respective regions within the region 70 causing a strong feeling of visual field interference, to perform the transparency-composition processing. For example, the transparency-composition unit 54 performs the transparency-composition processing at different composition ratios in the regions corresponding to the regions 71, 72, and 73. At this time, the transparency-composition unit 54 may perform the transparency composition in which the composition ratio of the image of the region within the region 70 that is closer to the position of the tip of the treatment instrument is increased. That is, the transparency-composition unit 54 may set the composition ratio of the image of the region 72 to be higher than the composition ratios of the images of the regions 71 and 73 to perform the transparency composition. As a result, the observer feels easier to recognize that the treatment instrument has appeared in the image. Note that, since the treatment instrument does not appear in the region 71, the observer does not feel the visual field interference. Therefore the region 71 may be excluded from the regions to be subjected to the transparency composition. In this case, the region to be subjected to the transparency composition may be reduced in size, and hence the processing speed may be increased. Further, when the size of the tip area of the treatment instrument that appears in the image does not satisfy a predetermined distance from the tip of the treatment instrument, the composition ratio of the region in which the treatment instrument appears may be set to a predetermined composition ratio. Note that, even when the tip of the treatment instrument is delivered beyond the region 70, the transparency composition may be performed so that the composition ratio of the image of the region within the region 70 that is closer to the position of the tip of the treatment instrument is increased. As a result, the observer feels easier to recognize the position and the track of the treatment instrument.

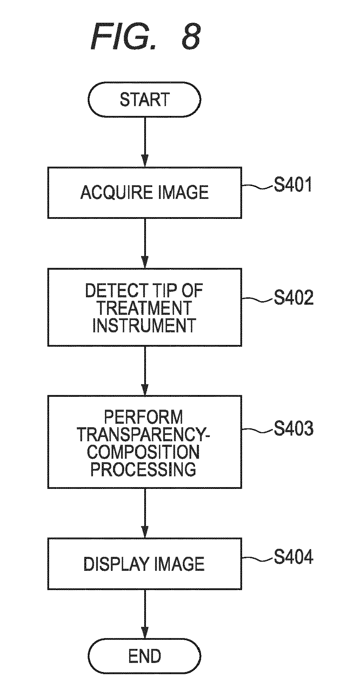

FIG. 8 is a flowchart of processing according to this embodiment. The processing of Steps S401 and S404 in FIG. 8 is similar to the processing of Steps S301 and S303 in the first embodiment, and description thereof is therefore omitted herein.

In Step S402, the tip-area detecting unit 53 detects the position of the tip of the treatment instrument within the image stored in the memory 51, and sends information on the detected tip area of the treatment instrument to the transparency-composition unit 54.

In Step S403, based on the position of the tip area of the treatment instrument that is sent from the tip-area detecting unit 53, the transparency-composition unit 54 controls the composition ratio of the image composition for the respective regions within the region 70 causing a strong feeling of visual field interference, to perform the transparency-composition processing. As a method of the transparency composition, the method described in the first embodiment is used.

In this embodiment, as described above, the position of the tip of the treatment instrument that appears in the picked-up image is detected, and based on the position of the tip area, the composition ratio of the image composition is controlled for the respective regions within the region causing a strong feeling of visual field interference, to perform the transparency-composition processing using the respective regions and the corresponding regions within the region having high correlation. Thus, it is possible to generate such an image that the treatment instrument becomes transparent in accordance with the composition ratios corresponding to the respective regions. Further, the transparency composition is performed so that the composition ratio of the image of the tip area is higher than the composition ratio of the image of the area other than the tip area. As a result, the observer feels easier to recognize that the treatment instrument has appeared through the channel in the image. Similarly, the transparency composition may be performed so as to set a higher composition ratio of the image of the region that is closer to the tip area. As a result, the observer feels easier to recognize the position and the track of the treatment instrument as well as the easiness of recognition that the treatment instrument has appeared through the channel in the image.

Third embodiment

In the first embodiment, the composition ratio of the transparency-composition processing is determined in advance. Alternatively, the observer may manually operate a slider switch and a volume switch to change the composition ratio in real time. Thus, in the initial stage of the operation of the treatment instrument, the observer may recognize the tip of the treatment instrument by increasing the composition ratio of the tip area of the treatment instrument within the observation screen, that is, by decreasing the transparency thereof. In the situation where the observer does not need to recognize the treatment instrument, the observer may decrease the composition ratio of the tip area of the treatment instrument, that is, increase the transparency thereof. In the case of inserting the treatment instrument through the channel, the observer needs to take care not to bring the treatment instrument into contact with an organ unintendedly, especially when delivering the tip of the treatment instrument from the tip of the endoscope. Therefore, it is effective that the observer may manually set the composition ratio freely depending on the situation so as to arbitrarily change the composition ratio at least in a part of the region causing a strong feeling of visual field interference. Further, in order that the observer may change the composition ratio of an arbitrary region within the observation screen, a touch panel may be mounted to the observation screen. With the touch panel, the observer may directly designate the size and position of the region to be subjected to the transparency composition. Further, a commercially-available gesture recognition system and the like may be used for the input (designation). As described above, the observer may arbitrarily set the composition ratio of an arbitrary region, and thus the observer feels easier to recognize the position and the track of the tip of the treatment instrument. Further, it can alleviate, for example, the feeling of visual field interference that is caused by sutures suspending the organ during a surgical operation, as well as the feeling of visual field interference that is caused by the treatment instrument.

### Fourth embodiment

In a fourth embodiment of the present invention, the control is performed in the following manner. That is, when the treatment instrument appears in the observation screen of the endoscope, the composition ratio of the region causing a strong feeling of visual field interference is set higher, and along with the elapse of time, the composition ratio is gradually decreased to a predetermined value, that is, the transparency of that region is increased to a predetermined value. Once the observer recognizes that the treatment instrument projects from the endoscope, the observer may recognize the treatment instrument even when the transparency is increased. The highest care needs to be taken when the treatment instrument appears in the image under a state in which the treatment instrument is completely absent from the observation screen. When the transparency is set to a high value at this time, there is a fear in that the observer is not aware that the treatment instrument has appeared, and the treatment instrument that has appeared may be brought into contact with the organ unintendedly. However, when the treatment instrument is retained for a long period of time in the region causing a strong feeling of visual field interference under a state in which the transparency is low, the observer cannot recognize the treatment instrument as a stereo image (cannot fuse the images), and hence the observer is liable to feel fatigue. Therefore, in the initial stage of the operation of the treatment instrument, the transparency is set lower so that the observer becomes aware that the treatment instrument has appeared, and along with the elapse of time, the transparency is increased. As a result, it is possible to alleviate the feeling of visual field interference while the observer is recognizing that the treatment instrument has appeared. Note that, the lower limit and the upper limit of the transparency may be set in advance, or the observer may set the transparency by using a keyboard, a touch panel, and the like while observing the image acquired through the endoscope. Further, the treatment instrument may be displayed as it is in a region in which visibility of the treatment instrument is important during a surgical operation and the like.

### Fifth embodiment

In the second embodiment, the template of the image of the treatment instrument is generated in advance, and the template matching is performed on the track of the treatment instrument, to thereby detect the position of the tip of the treatment instrument. However, the method of detecting the position of the tip of the treatment instrument is not limited to this method, and for example, there may also be used a method that involves installing, in the main body of the endoscope, a sensor for detecting the amount of delivery of the treatment instrument through the channel, and detecting the position of the tip of the treatment instrument based on information from the sensor. As the sensor, there may be used an optical sensor for optically detecting the movement amount, such as a sensor that is used in an optical mouse. Further, the search range of the template matching for detecting the position of the tip of the treatment instrument may be limited based on the information from the sensor. The position within the image at which the treatment instrument appears for the first time is uniquely determined, and hence, as a method realized without using the sensor, there may be used a method that involves constantly monitoring the above-mentioned position, and tracking the treatment instrument once the treatment instrument is detected.

### Sixth embodiment

In the first to fifth embodiments, as the image of the region to be combined, the image of the region causing a strong feeling of visual field interference is used as it is at the time of transparency-composition processing. In a sixth embodiment of the present invention, the image of the region causing a strong feeling of visual field interference is subjected to transformation processing so as to be converted into an image that is easier to observe, and the image is served for the transparency composition.

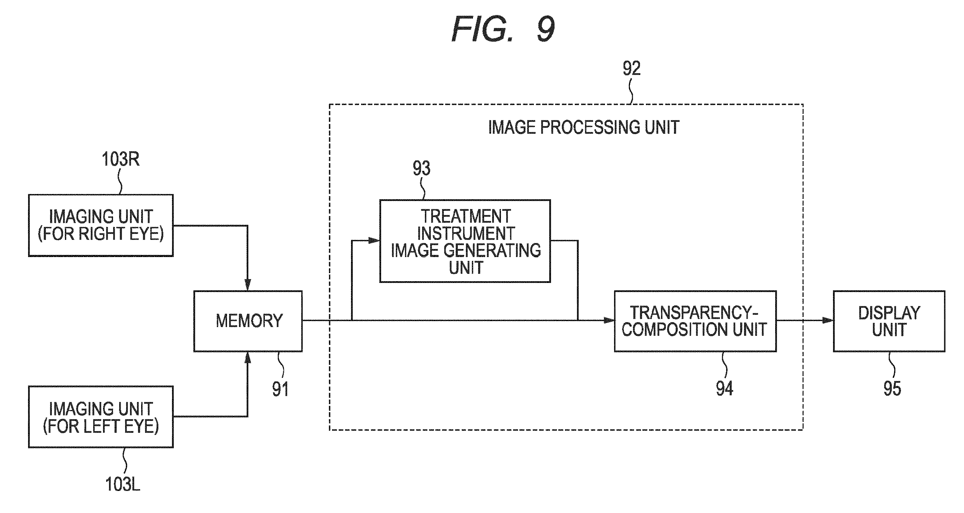

FIG. 9 is a functional block diagram of a processing system according to the sixth embodiment. FIG. 9 illustrates an imaging unit 103R for a right eye, an imaging unit 103L for a left eye, and a memory 91. Further, FIG. 9 illustrates an image processing unit 92. The image processing unit 92 includes a treatment instrument image generating unit 93 for generating treatment instrument images through transformation processing on the images picked up by the imaging units, and also includes a transparency-composition unit 94. FIG. 9 further illustrates a display unit 95. The image processing unit 92 in this case may be realized through use of, for example, a CPU. The imaging units 103R and 103L, the memory 91, and the display unit 95 are equivalent to those described in the first embodiment, and description thereof is therefore omitted herein.

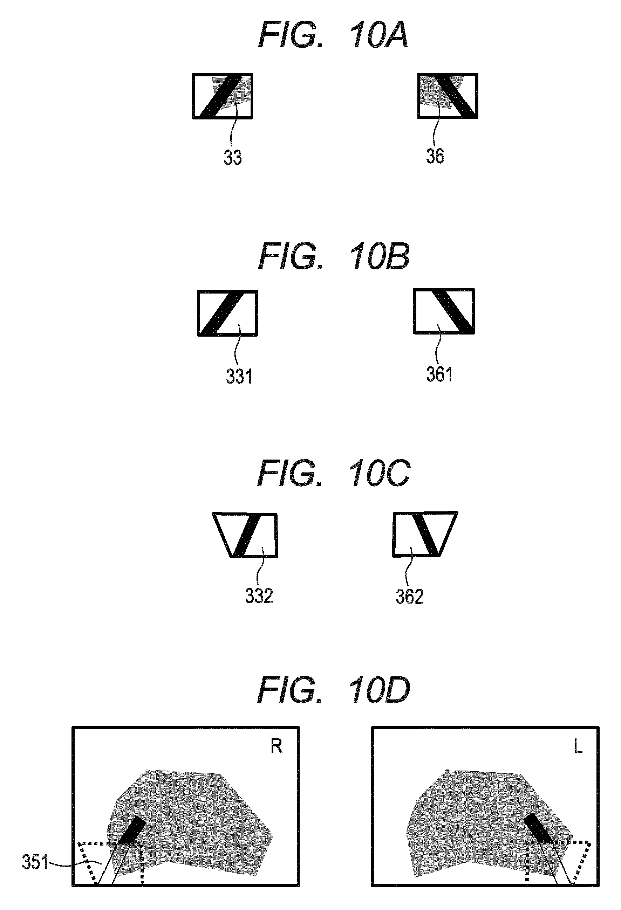

Referring to FIGS. 10A to 10D, an operation of the treatment instrument image generating unit 93 is described. FIGS. 10A to 10D schematically illustrate processing to be performed by the treatment instrument image generating unit 93. FIG. 10A illustrates the same regions as the region 33 illustrated in FIG. 3A and the region 36 illustrated in FIG. 3B. First, as illustrated in FIG. 10B, the treatment instrument image generating unit 93 generates images 331 and 361 by removing portions (hereinafter referred to as "backgrounds") other than the treatment instrument (or regions that the treatment instrument passes by) from the images of the regions 33 and 36 causing a strong feeling of visual field interference within the images for both eyes that are stored in the memory 91. Subsequently, as illustrated in FIG. 10C, the treatment instrument image generating unit 93 generates treatment instrument images 332 and 362 by transforming the images 331 and 361 so as to have a trapezoidal shape that is narrow in its bottom portion.

The transparency-composition unit 94 performs the transparency composition using the images 332 and 362 generated by the treatment instrument image generating unit 93, and on the images of the regions 34 and 37 having high correlation with the regions before the transformation. Thus, as illustrated in FIG. 10D, the transparency-composition unit 94 generates such an image that the parallax of the regions in which the treatment instrument appears is reduced and those regions become transparent in accordance with the composition ratio as in regions 351. Note that, the images of the regions 33 and 36 are transformed, and hence the edges of the images are lost, but in those portions, the images of the corresponding portions of the regions 34 and 37 having high correlation may be copied as it is without the transparency composition. Alternatively, the image 332 may be combined with the image of the region 34 by being superimposed thereon as it is without the transparency composition. Note that, in any case, the backgrounds of the images 332 and 362 become transparent so that the observer does not care about the backgrounds (the transparency of each background region is set to 1 or a value approximate thereto). Note that, at the time of transparency composition, the images of the transparent region within the images 332 and 362 may avoid being subjected to the transparency composition, and the corresponding areas of the regions 34 and 37 may be copied thereon and only the images of the regions in which the treatment instrument appears may be subjected to the transparency composition with the images of the regions 34 and 37.

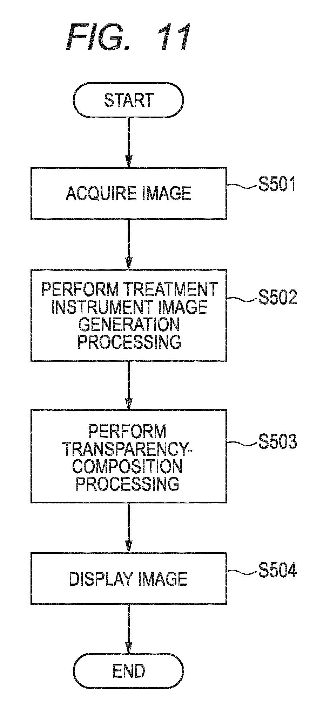

FIG. 11 is a flowchart of processing according to the sixth embodiment. The processing of Steps S501 and S504 is similar to the processing of Steps S301 and S303 in the first embodiment, and description thereof is therefore omitted herein.

In Step S502, the treatment instrument image generating unit 93 generates the treatment instrument images 332 and 362 by removing the background portions from the images of the regions causing a strong feeling of visual field interference within the images stored in the memory 91, and then transforming the resultant images so as to have a trapezoidal shape that is narrow in its bottom portion.

After the treatment instrument images 332 and 362 are generated, in Step S503, in the region causing a strong feeling of visual field interference within the image stored in the memory 91, the transparency-composition unit 94 performs the transparency composition using the treatment instrument images 332 and 362 and the images of the regions 34 and 37 at predetermined composition ratios, respectively.

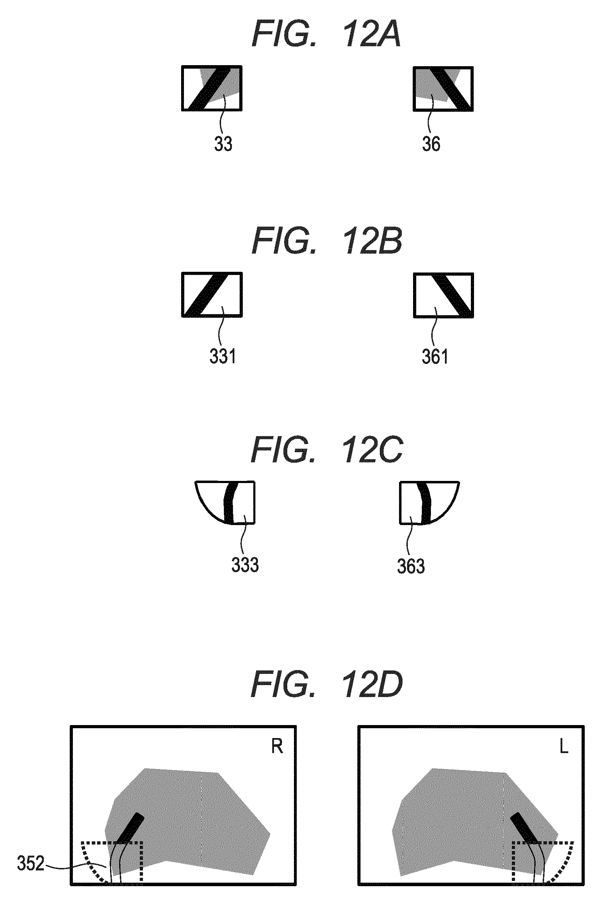

Alternatively, other processing may be applied in Step S502. FIGS. 12A to 12D schematically illustrate an example of this case. FIGS. 12A and 12B are illustrations equivalent to those of FIGS. 10A and 10B. As other processing to be applied in Step S502, for example, images 333 and 363 are generated by transforming the images 331 and 361 so as to have a curved surface as illustrated in FIG. 12C instead of transforming the images 331 and 361 so as to have a trapezoidal shape as illustrated in FIG. 10C. Then, the transparency composition is performed through use of the images 333 and 363. Thus, the treatment instrument portions appearing within the original images are smoothly connected to the treatment instrument portions having a curved surface through the transformation, thus alleviating a feeling of strangeness as compared to the case where the images are transformed so as to have a trapezoidal shape. FIG. 12D illustrates images obtained through the transparency-composition processing using the images resulting from the transformation processing.

In this embodiment, as described above, the treatment instrument images are generated by transforming the images of the regions causing a strong feeling of visual field interference so that the parallax is reduced, and the transparency composition is performed using the treatment instrument images and the images of the regions having high correlation with the regions causing a strong feeling of visual field interference. Thus, it is possible to provide a stereo image that is alleviated in the feeling of visual field interference and is easy to observe due to the reduction in parallax.

Seventh embodiment

In the sixth embodiment, at least a part of the picked-up images is transformed so that the parallax is reduced, and then the resultant images are served for the composition. In contrast, in a seventh embodiment of the present invention, a computer graphics (hereinafter referred to as "CG") technology is used for generating treatment instrument images that are easy to observe (that are reduced in parallax at the time of observation), and the resultant images are served for the transparency-composition processing.

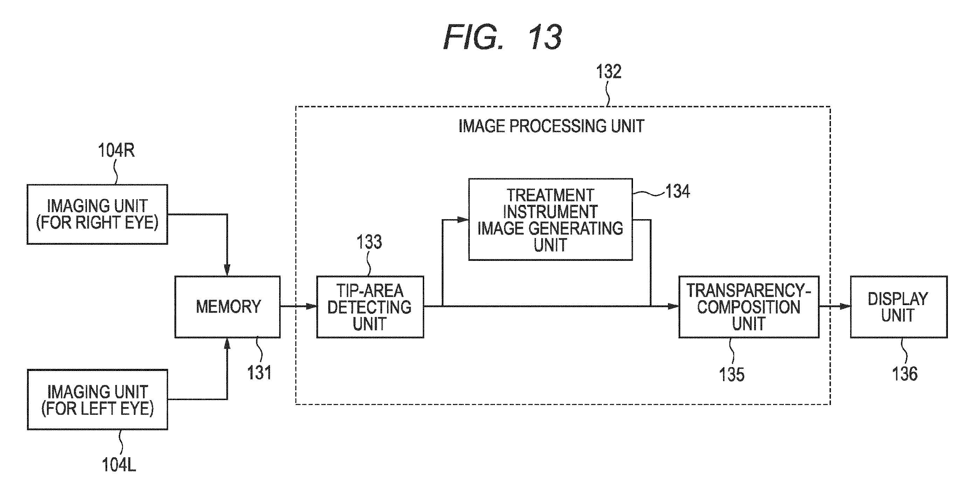

FIG. 13 is a functional block diagram of a processing system according to this embodiment. FIG. 13 illustrates an imaging unit 104R for a right eye, an imaging unit 104L for a left eye, and a memory 131. Further, FIG. 13 illustrates an image processing unit 132. The image processing unit 132 includes a tip-area detecting unit 133 for the treatment instrument, a treatment instrument image generating unit 134, and a transparency-composition unit 135. FIG. 13 further illustrates a display unit 136. The image processing unit 132 in this case may be realized through use of, for example, a CPU. The imaging units 104R and 104L, the memory 131, the tip-area detecting unit 133, and the display unit 136 are equivalent to those described in the second embodiment, and description thereof is therefore omitted herein.

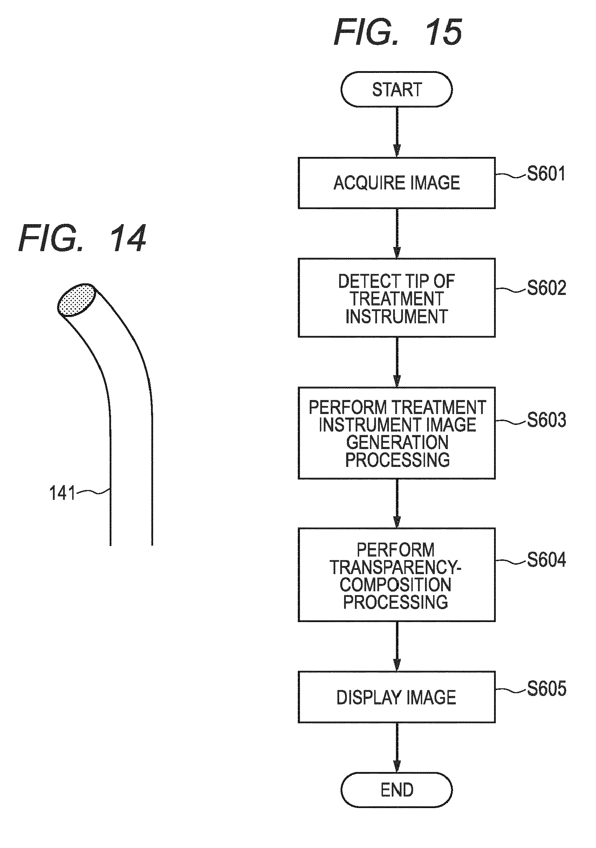

Referring to FIG. 14, an operation of the treatment instrument image generating unit 134 is described. FIG. 14 illustrates an example of a geometric model to be used in the CG technology when generating the treatment instrument images. A geometric model 141 is formed into a shape of a pipe having a curved tip portion. The geometric model 141 is rendered through shading under a condition that a light source and a viewpoint are set substantially equivalent to the optical system of the endoscope. In this manner, the treatment instrument images are generated. In this case, texture mapping or the like may be used instead of shading. After the treatment instrument images are generated, based on the position of the tip area of the treatment instrument that is detected by the tip-area detecting unit 133, there are identified portions of the treatment instrument images that correspond to the portion of the treatment instrument appearing in the regions causing a strong feeling of visual field interference. Note that, this identification processing may be performed by the transparency-composition unit.

Based on the position of the detected tip area, the transparency-composition unit 135 performs the transparency composition using the identified portions of the treatment instrument images and the images of the regions having high correlation with the regions causing a strong feeling of visual field interference. At this time, the level of the parallax on the treatment instrument images may be adjusted and the transparency composition may be performed so that the observer feels easy to observe the images.

FIG. 15 is a flowchart of processing according to this embodiment. The processing of Steps S601, S602, and S605 is similar to the processing of Steps S401, S402, and S404 in the second embodiment, and description thereof is therefore omitted herein.

In Step S603, the CG technology is used for rendering the geometric model 141 through shading under the condition that the light source and the viewpoint are set substantially equivalent to the optical system of the endoscope. In this manner, the treatment instrument images are generated. After the treatment instrument images are generated, based on the position of the tip area of the treatment instrument that is detected in Step S602, there are identified portions of the treatment instrument images that correspond to the portion of the treatment instrument appearing in the regions causing a strong feeling of visual field interference.

Subsequently, in Step S604, in the regions causing a strong feeling of visual field interference within the images for both eyes that are stored in the memory 131, based on the position of the detected tip area of the treatment instrument, the transparency-composition unit 135 performs the transparency composition using the identified portions of the treatment instrument images and the images of the regions having high correlation with the regions causing a strong feeling of visual field interference.

In this embodiment, as described above, instead of using the actual images, the treatment instrument images generated through use of the CG technology are subjected to the transparency-composition processing with the images of the regions having high correlation with the regions causing a strong feeling of visual field interference at predetermined composition ratios, respectively, based on the position of the tip of the treatment instrument within the picked-up images. Thus, it is possible to provide a stereo image that is alleviated in the feeling of visual field interference and is easy to observe due to the reduction in parallax.

Eighth embodiment

In the first embodiment, the transparency composition is performed directly using the image of the region causing a strong feeling of visual field interference and the image of the region having high correlation therewith. In an eighth embodiment of the present invention, the treatment instrument is deleted from the region causing a strong feeling of visual field interference once, and then the transparency-composition processing is performed.

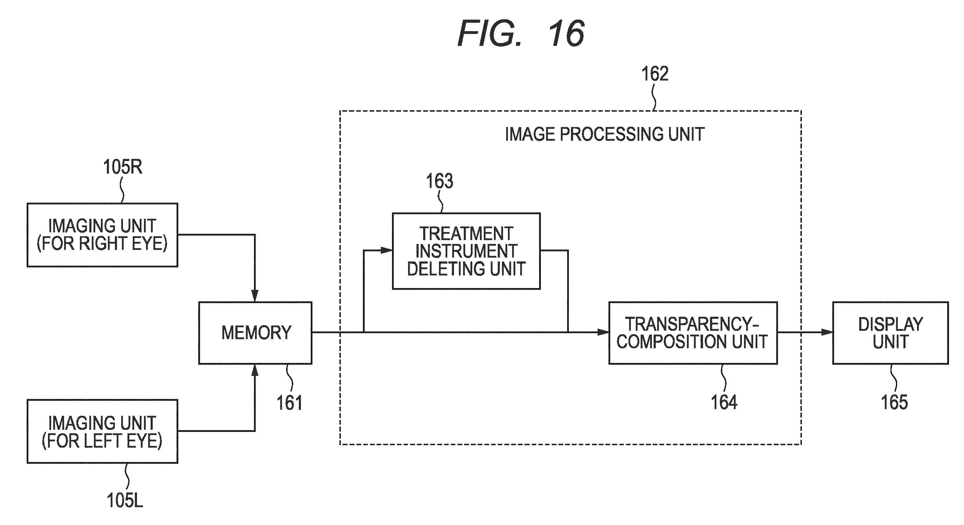

FIG. 16 is a functional block diagram of a processing system according to this embodiment. FIG. 16 illustrates an imaging unit 105R for a right eye, an imaging unit 105L for a left eye, and a memory 161. Further, FIG. 16 illustrates an image processing unit 162 for processing images. The image processing unit 162 includes a treatment instrument deleting unit 163 for deleting the treatment instrument from the images picked up by the imaging units, and also includes a transparency-composition unit 164. FIG. 16 further illustrates a display unit 165. The image processing unit 162 in this case may be realized through use of, for example, a CPU. The imaging units 105R and 105L, the memory 161, and the display unit 165 are equivalent to those described in the first embodiment, and description thereof is therefore omitted herein.

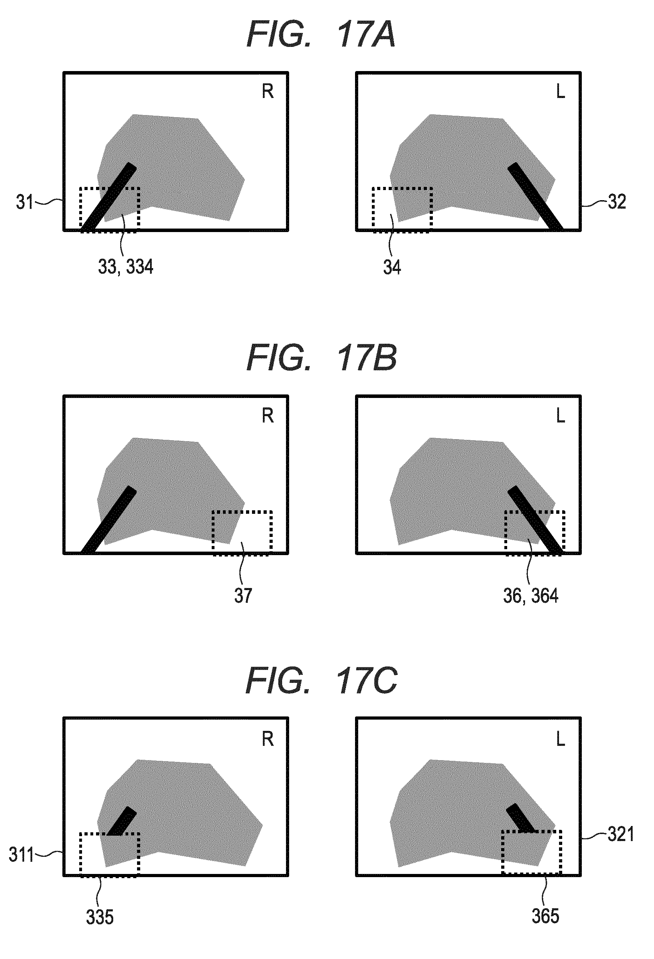

Referring to FIGS. 17A to 17C, processing to be performed by the treatment instrument deleting unit 163 is described. First, as illustrated in FIG. 17A, similarly to the first embodiment, the region (first region) 33 causing a strong feeling of visual field interference is determined in advance within the image (first image) 31 for the right eye. At this time, the image of the region 33 causing a strong feeling of visual field interference is stored in the memory 161 as an image 334. Subsequently, the region (second region) 34 having high correlation with the region 33 is identified within the image (second image) 32 for the left eye. As a method of identifying the region 34, the region-based matching or the like may be used as described in the first embodiment. After the region 34 having high correlation is identified, the treatment instrument deleting unit 163 copies the image of the region 34 onto the region 33 within the image 31, and thus, as illustrated in FIG. 17C, the treatment instrument deleting unit 163 may generate an image (third image) 311 in which the treatment instrument is deleted in a region 335. Further, similar processing is performed on the image 32 for the left eye. That is, as illustrated in FIG. 17B, the region 36 causing a strong feeling of visual field interference is determined within the image 32 for the left eye, and the image of the region 36 is stored in the memory as an image 364. Subsequently, the region 37 having high correlation with the region 36 is identified within the image 31 for the right eye. Then, the treatment instrument deleting unit 163 copies the image of the region 37 onto the region 36 within the image 32, and thus, as illustrated in FIG. 17C, the treatment instrument deleting unit 163 may generate an image 321 in which the treatment instrument is deleted in a region 365.

Next, processing to be performed by the transparency-composition unit 164 is described. In the region (third region) 335 in which the region 34 is copied within the image 311 (within the third image) in which the treatment instrument is deleted, the transparency-composition unit 164 performs the transparency composition using the image of the region 335 and the image 334 of the region causing a strong feeling of visual field interference, which is stored in the memory 161. Similarly, for the image for the left eye, in the region 365 in which the treatment instrument is deleted within the image 321, the transparency-composition unit 164 performs the transparency composition using the image of the region 365 and the image 364 of the region causing a strong feeling of visual field interference, which is stored in the memory 161. Thus, as illustrated in FIG. 3C, the transparency-composition unit 164 may generate such an image that the treatment instrument becomes transparent in accordance with the composition ratio in the region causing a strong feeling of visual field interference, thereby alleviating the feeling of visual field interference.

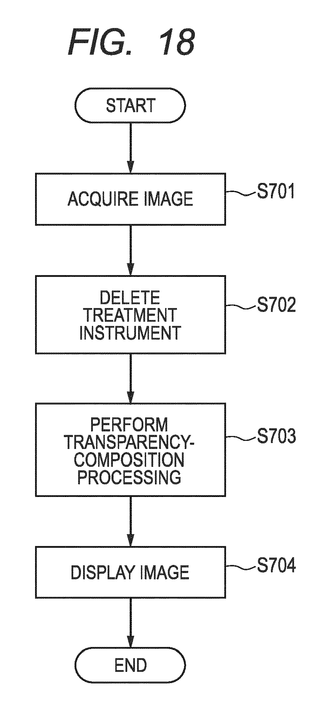

FIG. 18 is a flowchart of processing according to this embodiment. The processing of Steps S701 and S704 is similar to the processing of Steps S301 and S304 in the first embodiment, and description thereof is therefore omitted herein.

In Step S702, within the images for both eyes that are stored in the memory 161, the images 334 and 364 of the regions causing a strong feeling of visual field interference are stored in the memory 161. Further, onto the regions causing a strong feeling of visual field interference within the images for both eyes, the treatment instrument deleting unit 163 copies the images of the regions having high correlation with those regions within the images from the respective opposite eyes. Thus, the treatment instrument deleting unit 163 generates the images 311 and 321 in which the treatment instrument is deleted from the regions causing a strong feeling of visual field interference.

Subsequently, in Step S703, in the regions 335 and 365 in which the treatment instrument is deleted within the images 311 and 321, the transparency-composition unit 164 performs the transparency composition using the images of the regions 335 and 365 and the original images 334 and 364 of the regions causing a strong feeling of visual field interference, which are stored in the memory 161, respectively.

In this embodiment, as described above, onto the region causing a strong feeling of visual field interference, the image of the region having high correlation therewith, in which the treatment instrument does not appear, is copied. Thus, there is generated an image in which the treatment instrument is deleted from the region causing a strong feeling of visual field interference. After that, the transparency composition is performed using the image of the region in which the treatment instrument is deleted and the original image of the region causing a strong feeling of visual field interference, in which the treatment instrument appears. Thus, it is possible to provide such an image that the treatment instrument becomes transparent in accordance with the composition ratio. Therefore, it is possible to alleviate the feelings of visual field interference and fatigue while the observer is recognizing the treatment instrument.

Ninth embodiment

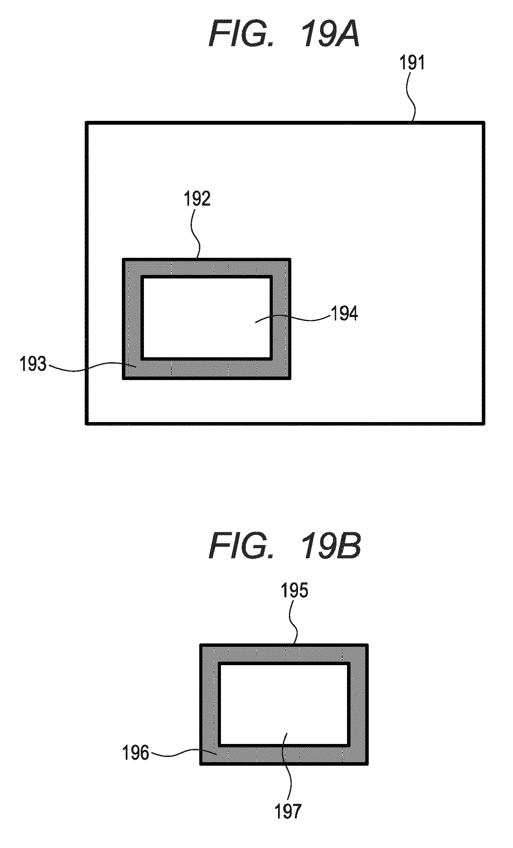

In the first embodiment, when the transparency composition is performed using the image of the region causing a strong feeling of visual field interference and the image of the region having high correlation therewith, due to influence of, for example, a brightness difference between the image of the region that is subjected to the transparency composition and the image of the region that is not subjected to the transparency composition, an edge may be generated at a junction (boundary) portion between those regions. In a ninth embodiment of the present invention, to address this problem, when performing the transparency composition using the image of the region causing a strong feeling of visual field interference and the image of the region having high correlation therewith, the transparency composition is performed in a boundary region having a predetermined width (about 1/10 of the size of the image) so that the transparency of the image of the region causing a strong feeling of visual field interference is gradually increased along with increase in distance from a boundary of that region. Thus, it is possible to obtain an inconspicuous boundary portion between the region that is subjected to the transparency composition and the region that is not subjected to the transparency composition. FIGS. 19A and 19B illustrate a state of processing to be performed in this case. An image 191 corresponds to the image 31 described in the first embodiment; a region 192 corresponds to the region 33; and a region 195 corresponds to the region 34. Further, FIG. 19A illustrates a boundary region 193 of the region 192, and a region (center region) 194 defined by removing the boundary region 193 from the region 192. Similarly, FIG. 19B illustrates a boundary region 196 of the region 195, and a region (center region) 197 defined by removing the boundary region 196 from the region 195. In this embodiment, when performing the transparency composition using the image of the region 192 and the image of the region 195, the transparency composition is performed in the boundary region 193 so that the composition ratio of the image of the boundary region 193 is gradually decreased from a predetermined value along with increase in distance from a boundary of the region 192 toward the center region 194 thereof. In other words, the transparency composition is performed using the image of the boundary region 193 and the image of the boundary region 196 so that the transparency of the boundary region 193 is increased from a predetermined value to a value of, for example, the transparency of the center region 194 along with increase in distance from the boundary of the region 192 toward the center region 194 thereof. Thus, it is possible to obtain an even more inconspicuous boundary portion as compared to the case where the transparency composition is simply performed using the entire image of the region 192 at a predetermined composition ratio.

Further, in a case where this embodiment is combined with the eighth embodiment, the above-mentioned processing of obtaining an inconspicuous edge may be performed when copying the image of the region 195 having high correlation onto the region 192 causing a strong feeling of visual field interference, instead of when performing the transparency-composition processing. Specifically, when copying the image of the region 195 having high correlation, the transparency composition is performed on the image of the boundary region 193 and the image of the boundary region 196 so that the composition ratio of the boundary region 193 is gradually decreased from the predetermined value along with increase in distance from the boundary of the region 192 toward the center region 194 thereof. For the center region, the center region 197 is copied onto the center region 194. After that, the transparency composition is performed using the image of the center region that is obtained after the image of the center region 197 is copied and the treatment instrument is therefore deleted and the image of the center region 194 that is stored in advance before the treatment instrument is deleted. Thus, it is possible to obtain an even more inconspicuous boundary portion as compared to the case where the image is simply copied.

Tenth embodiment

In the eighth embodiment, the treatment instrument deletion processing is performed on the regions causing a strong feeling of visual field interference within the images for both eyes, and then the transparency composition is performed on the images of the regions from which the treatment instrument is deleted and the images of the regions in which the treatment instrument appears, respectively. In a tenth embodiment of the present invention, the treatment instrument deletion processing is performed only on any one of the image for the left eye and the image for the right eye, and the transparency-composition processing is performed only on the image for the other eye. Alternatively, the treatment instrument deletion processing may be performed on the images for both eyes, and then the transparency composition may be performed only on any one of the image for the left eye and the image for the right eye. Note that, at this time, similarly to the first embodiment, the composition ratio of the transparency composition may be set arbitrarily, and therefore the transparency of the treatment instrument may be set arbitrarily. Thus, the regions in which the observer feels difficult to fuse images are displayed as a two-dimensional image, and the treatment instrument becomes transparent in accordance with the composition ratio. Therefore, the observer may recognize the images as a stereo image in the substantially entire region, and may focus on the background without stress. As a result, it is possible to alleviate the feeling of visual field interference. Alternatively, the treatment instrument deletion processing may be performed only on any one of the image for the left eye and the image for the right eye, and the transparency composition may be omitted on the image for the other eye too. Further, the treatment instrument deletion processing may be performed on the images for both eyes, and the image of the region causing a strong feeling of visual field interference may be copied only onto any one of the image for the left eye and the image for the right eye. Also in any of the above-mentioned cases where the transparency composition is omitted, the regions in which the observer feels difficult to fuse images are displayed as a two-dimensional image, and in the other regions, the observer may recognize the images as a stereo image. Therefore, the observer may focus on the background without stress. As a result, it is possible to alleviate the feeling of visual field interference. Thus, it is possible to alleviate the feelings of visual field interference and fatigue while the observer is recognizing the treatment instrument. Further, the processing of the ninth embodiment may be performed when performing the treatment instrument deletion processing or the transparency-composition processing, and hence an inconspicuous boundary region is obtained. Thus, it is possible to provide an image that is easier to observe the treatment instrument.

Further, the methods used in the respective embodiments may be applied in combination.

Other Embodiments

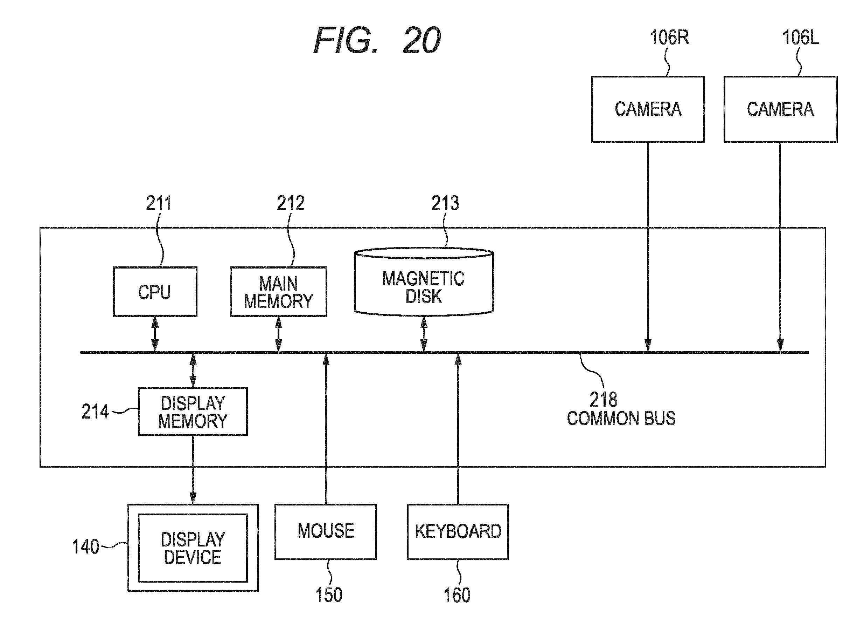

In the respective embodiments described above, the processing may be performed through use of a control box or the like (not shown) of the endoscope apparatus. Further, embodiments of the present invention can also be realized by a computer of a system or apparatus that reads out and executes computer executable instructions recorded on a storage medium (e.g., non-transitory computer-readable storage medium) to perform the functions of one or more of the above-described embodiment(s) of the present invention, and by a method performed by the computer of the system or apparatus by, for example, reading out and executing the computer executable instructions from the storage medium to perform the functions of one or more of the above-described embodiment(s). The computer may comprise one or more of a central processing unit (CPU), micro processing unit (MPU), or other circuitry, and may include a network of separate computers or separate computer processors. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)™), a flash memory device, a memory card, and the like. For example, a computer having a configuration as illustrated in FIG. 20 may be used for realizing similar processing. FIG. 20 illustrates a CPU 211, a main memory 212, a magnetic disk 213, a display memory 214, a common bus 218, a display device 140, a mouse 150, and a keyboard 160. Further, FIG. 20 illustrates imaging units (cameras) 106R and 106L of the stereo endoscope.

The respective embodiments described above are directed to the stereo endoscope apparatus, but it is understood that the scope of the present invention to be applied is not limited to the stereo endoscope apparatus. For example, the methods used in the respective embodiments may also be applied in a case where a general stereo camera is used for picking up an image of a landscape but a subject is present in a region causing a strong feeling of visual field interference (present in proximity to the camera). In other words, it is possible to alleviate the feeling of visual field interference due to the subject through the transparency-composition processing according to the respective embodiments.

As described above, according to the respective embodiments, in the region causing a strong feeling of visual field interference, the image in which the treatment instrument appears and the image in which the treatment instrument does not appear are subjected to transparency composition at a predetermined composition ratio. Thus, the treatment instrument becomes transparent in accordance with the predetermined composition ratio. Therefore, it is possible to alleviate the feelings of visual field interference and fatigue while the observer is recognizing the treatment instrument. Further, according to the respective embodiments, similar effects may be attained in a stereo imaging and displaying system as well as the stereo endoscope.

Thus, according to the present invention, a predetermined region having parallax at a high level becomes transparent in accordance with the composition ratio, and hence it is possible to provide a stereo image that is alleviated in the feeling of visual field interference.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

Provided is a stereo endoscope apparatus, including: a treatment instrument that is operable; at least two imaging units for picking up images of a subject; and a transparency-composition unit for performing, in a first region in which the treatment instrument appears within a first image picked up by one of the at least two imaging units, image composition by blending, at a predetermined composition ratio, an image of the first region and an image of a second region in which the treatment instrument does not appear within a second image picked up by another of the at least two imaging units.

## Claims

1. A stereo endoscope apparatus, comprising:

   a treatment instrument that is operable;
   at least two imaging units arranged to pick up images of a subject; and
   a transparency-composition unit configured to perform, in a first region in which the treatment instrument appears within a first image picked up by one of the at least two imaging units, image composition by blending, at a predetermined composition ratio, an image of the first region and an image of a second region in which the treatment instrument does not appear within a second image picked up by another of the at least two imaging units.

2. A stereo endoscope apparatus according to claim 1, wherein the transparency-composition unit changes a composition ratio of the image of the first region at least in a part of the first region.

3. A stereo endoscope apparatus according to claim 2, further comprising a tip-area detecting unit configured to detect a tip area of the treatment instrument within the first image,
   wherein the transparency-composition unit changes the composition ratio of the image of the first region at least in the part of the first region based on a position of the detected tip area.

4. A stereo endoscope apparatus according to any one of claims 1 to 3, wherein at least one of a size and a position of the first region is controlled to be changed.

5. A stereo endoscope apparatus according to any one of claims 1 to 4, wherein the transparency-composition unit changes the composition ratio of the image of the first region so as to be gradually decreased to a predetermined value along with elapse of time.

6. A stereo endoscope apparatus according to any one of claims 1 to 5, further comprising a treatment instrument deleting unit configured to generate a third image by copying the image of the second region onto the first region within the first image, wherein, in a third region in which the image of the second region is copied within the third image, the transparency-composition unit performs the image composition by blending an image of the third region and the image of the first region at the predetermined composition ratio.

7. A stereo endoscope apparatus according to any one of claims 1 to 6, wherein the transparency-composition unit performs the image composition so that a composition ratio of an image of a boundary region of the first region is gradually decreased from a predetermined value along with increase in distance from a boundary of the first region toward a center region thereof.

8. A stereo endoscope apparatus according to claim 6, wherein, when generating the third image, the treatment instrument deleting unit generates an image of a boundary region of the third region through image composition performed by blending, in a boundary region of the first region, an image of the boundary region of the first region and an image of a boundary region of the second region so that a composition ratio of the image of the boundary region of the first region is gradually decreased from a predetermined value along with increase in distance from a boundary of the first region toward a center region thereof.

9. A stereo endoscope apparatus, comprising:

a treatment instrument that is operable;
at least two imaging units arranged to pick up images of a subject;
a treatment instrument image generating unit configured to generate a treatment instrument image by transforming a part of an image of a first region in which the treatment instrument appears within a first image picked up by one of the at least two imaging units; and
a transparency-composition unit configured to perform, in the first region within the first image, image composition by blending, at a predetermined composition ratio, the treatment instrument image and an image of a second region in which the treatment instrument does not appear within a second image picked up by another of the at least two imaging units.

10. A stereo endoscope apparatus, comprising:

a treatment instrument that is operable;
at least two imaging units arranged to pick up images of a subject;
a treatment instrument image generating unit configured to generate a treatment instrument image through use of a computer graphics technology; and
a transparency-composition unit configured to perform, in a first region in which the treatment instrument appears within a first image picked up by one of the at least two imaging units, image composition by blending, at a predetermined composition ratio, the treatment instrument image and an image of a second region in which the treatment instrument does not appear within a second image picked up by another of the at least two imaging units.

11. An image processing method for a stereo endoscope apparatus comprising a treatment instrument that is operable, the image processing method comprising:

picking up images of a subject by at least two imaging units; and
performing, in a first region in which the treatment instrument appears within a first image picked up by one of the at least two imaging units, image composition by blending, at a predetermined composition ratio, an image of the first region and an image of a second region in which the treatment instrument does not appear within a second image picked up by another of the at least two imaging units.

12. An image processing method according to claim 11, further comprising changing a composition ratio of the image of the first region at least in a part of the first region.

13. An image processing method according to claim 12, further comprising:

detecting a tip area of the treatment instrument within the first image; and
changing the composition ratio of the image of the first region at least in the part of the first region based on a position of the detected tip area.

14. An image processing method according to any one of claims 11 to 13, further comprising changing at least one of a size and a position of the first region.

**15.** An image processing method according to any one of claims 11 to 14, further comprising changing the composition ratio of the image of the first region so as to be gradually decreased to a predetermined value along with elapse of time.

**16.** An image processing method according to any one of claims 11 to 15, further comprising generating a third image by copying the image of the second region onto the first region within the first image, wherein the performing image composition comprises blending, in a third region in which the image of the second region is copied within the third image, an image of the third region and the image of the first region at the predetermined composition ratio.

**17.** An image processing method according to any one of claims 11 to 16, wherein the performing image composition comprises performing the image composition so that a composition ratio of an image of a boundary region of the first region is gradually decreased from a predetermined value along with increase in distance from a boundary of the image of the first region toward a center region thereof.

**18.** An image processing method according to claim 16, wherein the generating a third image comprises generating an image of a boundary region of the third region through image composition performed by blending, in a boundary region of the first region, an image of the boundary region of the first region and an image of a boundary region of the second region so that a composition ratio of the image of the boundary region of the first region is gradually decreased from a predetermined value along with increase in distance from a boundary of the first region toward a center region thereof.

**19.** An image processing method for a stereo endoscope apparatus comprising a treatment instrument that is operable, the image processing method comprising:

picking up images of a subject by at least two imaging units;
generating a treatment instrument image by transforming at least a part of an image of a first region in which the treatment instrument appears within a first image picked up by one of the at least two imaging units; and
performing, in the first region within the first image, image composition by blending, at a predetermined composition ratio, the treatment instrument image and an image of a second region in which the treatment instrument does not appear within a second image picked up by another of the at least two imaging units.

**20.** An image processing method for a stereo endoscope apparatus comprising a treatment instrument that is operable, the image processing method comprising:

picking up images of a subject by at least two imaging units;
generating a treatment instrument image through use of a computer graphics technology; and
performing, in a first region in which the treatment instrument appears within a first image picked up by one of the at least two imaging units, image composition by blending, at a predetermined composition ratio, the treatment instrument image and an image of a second region in which the treatment instrument does not appear within a second image picked up by another of the at least two imaging units.

# FIG. 1

101R

IMAGING UNIT
(FOR RIGHT EYE)

IMAGING UNIT
(FOR LEFT EYE)

101L

MEMORY

11

12

IMAGE PROCESSING UNIT

TRANSPARENCY-
COMPOSITION UNIT

13

DISPLAY
UNIT

14

# FIG. 2

100

101R

101L

24

25

23

## FIG. 3A

## FIG. 3B

## FIG. 3C

# FIG. 4

```
        ( START )
            │
            ▼
┌─────────────────────┐
│    ACQUIRE IMAGE    │——S301
└─────────────────────┘
            │
            ▼
┌─────────────────────┐
│      PERFORM        │
│   TRANSPARENCY-     │——S302
│    COMPOSITION      │
│    PROCESSING       │
└─────────────────────┘
            │
            ▼
┌─────────────────────┐
│    DISPLAY IMAGE    │——S303
└─────────────────────┘
            │
            ▼
        ( END )
```

FIG. 5

EP 2 762 057 A1

*FIG. 6A*

*FIG. 6B*

*FIG. 6C*

## FIG. 7A

R

70

## FIG. 7B

R

71

72

73

# FIG. 8

```
        ┌──────────┐
        │  START   │
        └──────────┘
              │
              ▼
   ┌─────────────────────┐
   │   ACQUIRE IMAGE     │────S401
   └─────────────────────┘
              │
              ▼
   ┌─────────────────────┐
   │   DETECT TIP OF     │
   │   TREATMENT         │────S402
   │   INSTRUMENT        │
   └─────────────────────┘
              │
              ▼
   ┌─────────────────────┐
   │   PERFORM           │
   │   TRANSPARENCY-     │
   │   COMPOSITION       │────S403
   │   PROCESSING        │
   └─────────────────────┘
              │
              ▼
   ┌─────────────────────┐
   │   DISPLAY IMAGE     │────S404
   └─────────────────────┘
              │
              ▼
        ┌──────────┐
        │   END    │
        └──────────┘
```

FIG. 9

IMAGE PROCESSING UNIT 92

TREATMENT INSTRUMENT IMAGE GENERATING UNIT 93

TRANSPARENCY-COMPOSITION UNIT 94

DISPLAY UNIT 95

MEMORY 91

IMAGING UNIT (FOR RIGHT EYE) 103R

IMAGING UNIT (FOR LEFT EYE) 103L

## FIG. 10A

33

36

## FIG. 10B

331

361

## FIG. 10C

332

362

## FIG. 10D

R

L

351

# FIG. 11

```
        ┌─────────────┐
        │    START    │
        └─────────────┘
               │
               ▼
    ┌─────────────────────┐
    │    ACQUIRE IMAGE     │────── S501
    └─────────────────────┘
               │
               ▼
    ┌─────────────────────┐
    │  PERFORM TREATMENT   │
    │  INSTRUMENT IMAGE    │────── S502
    │     GENERATION       │
    │     PROCESSING       │
    └─────────────────────┘
               │
               ▼
    ┌─────────────────────┐
    │      PERFORM         │
    │   TRANSPARENCY-      │
    │    COMPOSITION       │────── S503
    │     PROCESSING       │
    └─────────────────────┘
               │
               ▼
    ┌─────────────────────┐
    │    DISPLAY IMAGE     │────── S504
    └─────────────────────┘
               │
               ▼
        ┌─────────────┐
        │     END     │
        └─────────────┘
```

FIG. 12A

33

36

FIG. 12B

331

361

FIG. 12C

333

363

FIG. 12D

R

L

352

*FIG. 13*

IMAGE PROCESSING UNIT — 132

IMAGING UNIT (FOR RIGHT EYE) — 104R

IMAGING UNIT (FOR LEFT EYE) — 104L

MEMORY — 131

TIP-AREA DETECTING UNIT — 133

TREATMENT INSTRUMENT IMAGE GENERATING UNIT — 134

TRANSPARENCY-COMPOSITION UNIT — 135

DISPLAY UNIT — 136

## FIG. 15

START

ACQUIRE IMAGE ⎯S601

DETECT TIP OF TREATMENT INSTRUMENT ⎯S602

PERFORM TREATMENT INSTRUMENT IMAGE GENERATION PROCESSING ⎯S603

PERFORM TRANSPARENCY-COMPOSITION PROCESSING ⎯S604

DISPLAY IMAGE ⎯S605

END

## FIG. 14

141

*FIG. 16*

EP 2 762 057 A1

## FIG. 17A

R
L
31
32
33, 334
34

## FIG. 17B

R
L
37
36, 364

## FIG. 17C

R
L
311
321
335
365

# FIG. 18

```
        ( START )
            |
            v
  +-------------------+
  |   ACQUIRE IMAGE   |——S701
  +-------------------+
            |
            v
  +-------------------+
  |      DELETE       |
  |     TREATMENT     |——S702
  |    INSTRUMENT     |
  +-------------------+
            |
            v
  +-------------------+
  |     PERFORM       |
  |   TRANSPARENCY-   |
  |   COMPOSITION     |——S703
  |    PROCESSING     |
  +-------------------+
            |
            v
  +-------------------+
  |   DISPLAY IMAGE   |——S704
  +-------------------+
            |
            v
        (  END  )
```

## FIG. 19A

191

192

194

193

## FIG. 19B

195

196

197

FIG. 20

EP 2 762 057 A1

Europäisches
Patentamt

European
Patent Office

Office européen
des brevets

## EUROPEAN SEARCH REPORT

Application Number

EP 14 15 2982

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2012/271102 A1 (KATAYAMA AKIHIRO [JP]) 25 October 2012 (2012-10-25) | 1-9, 11-19 | INV. A61B1/00 G02B23/24 H04N13/00 H04N13/02 |
| Y | * figure 1 * <br> * figure 2 * <br> * paragraph [0025] * <br> * figure 3 * <br> * paragraph [0038] * <br> ----- | 10,20 | |
| Y | US 2011/306986 A1 (LEE MIN KYU [KR] ET AL) 15 December 2011 (2011-12-15) <br> * figure 6 * <br> * figure 7 * <br> * figure 8 * <br> ----- | 10,20 | |
| A | TAIKI FUKIAGE ET AL: "Reduction of contradictory partial occlusion in mixed reality by using characteristics of transparency perception", MIXED AND AUGMENTED REALITY (ISMAR), 2012 IEEE INTERNATIONAL SYMPOSIUM ON, IEEE, 5 November 2012 (2012-11-05), pages 129-139, XP032309059, DOI: 10.1109/ISMAR.2012.6402549 ISBN: 978-1-4673-4660-3 <br> * figure 1 * <br> * figure 3 * <br> * figure 6 * <br> * figure 9 * <br> * abstract * <br> ----- <br> -/-- | 2,3 | TECHNICAL FIELDS SEARCHED (IPC) <br><br> H04N <br> G03B <br> G06T <br> A61B <br> G02B |

The present search report has been drawn up for all claims

| Place of search | Date of completion of the search | Examiner |
|---|---|---|
| The Hague | 11 April 2014 | Benzeroual, Karim |

EPO FORM 1503 03.82 (P04C01)

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | JP 2004 065804 A (FUJI PHOTO OPTICAL CO LTD) 4 March 2004 (2004-03-04)<br>* paragraph [0001] *<br>* figure 2A *<br>* paragraph [0016] *<br>* figure 2B *<br>* figure 2C *<br>* figure 8A *<br>* figure 8B *<br>* example 1 *<br>* paragraph [0024] *<br>* figure 4A *<br>* figure 4B *<br>* figure 4C *<br>* paragraph [0022] * | 1-9,<br>11-19<br>10,20 | |
| A | JP 2004 187711 A (OLYMPUS CORP) 8 July 2004 (2004-07-08)<br>* figure 10 *<br>* figure 13 *<br>* figure 16 *<br>* figure 17 * | 1-20 | |
| A | WO 2006/087663 A1 (KONINKL PHILIPS ELECTRONICS NV [NL]; VISSER HUGO [FR]; SALGO IVAN [FR]) 24 August 2006 (2006-08-24)<br>* figure 3 *<br>* figure 4 *<br>* figure 5 * | 1-20 | |
| A | US 2007/008404 A1 (TOMITA SEIJIRO [JP]) 11 January 2007 (2007-01-11)<br>* figure 1 *<br>* figure 2 *<br>* figure 4 *<br>* figure 5 * | 1,9-11,<br>19,20 | |

TECHNICAL FIELDS
SEARCHED        (IPC)

-/--

The present search report has been drawn up for all claims

| Place of search | Date of completion of the search | Examiner |
|---|---|---|
| The Hague | 11 April 2014 | Benzeroual, Karim |

EPO FORM 1503 03.82 (P04C01)

Europäisches
Patentamt

European
Patent Office

Office européen
des brevets

# EUROPEAN SEARCH REPORT

Application Number

EP 14 15 2982

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2009/268010 A1 (ZHAO WENYI [US] ET AL) 29 October 2009 (2009-10-29)<br>* paragraph [0013] *<br>* paragraph [0024] *<br>* figure 15 *<br>----- | 1-20 | |
| A | US 2012/062560 A1 (SWOBODA CORNEL [DE] ET AL) 15 March 2012 (2012-03-15)<br>* paragraph [0058] *<br>* paragraph [0015] *<br>----- | 7,8,17, 18 | |

TECHNICAL FIELDS
SEARCHED (IPC)

The present search report has been drawn up for all claims

| Place of search | Date of completion of the search | Examiner |
|---|---|---|
| The Hague | 11 April 2014 | Benzeroual, Karim |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 14 15 2982

This annex lists the patent family members relating to the patent documents cited in the above-mentioned European search report.
The members are as contained in the European Patent Office EDP file on
The European Patent Office is in no way liable for these particulars which are merely given for the purpose of information.

11-04-2014

| Patent document cited in search report | | Publication date | Patent family member(s) | | Publication date |
|---|---|---|---|---|---|
| US 2012271102 | A1 | 25-10-2012 | JP | 2012223446 A | 15-11-2012 |
| | | | US | 2012271102 A1 | 25-10-2012 |
| US 2011306986 | A1 | 15-12-2011 | CN | 102341046 A | 01-02-2012 |
| | | | US | 2011306986 A1 | 15-12-2011 |
| | | | WO | 2010110560 A2 | 30-09-2010 |
| JP 2004065804 | A | 04-03-2004 | JP | 4170042 B2 | 22-10-2008 |
| | | | JP | 2004065804 A | 04-03-2004 |
| JP 2004187711 | A | 08-07-2004 | NONE | | |
| WO 2006087663 | A1 | 24-08-2006 | EP | 1854303 A1 | 14-11-2007 |
| | | | JP | 4860636 B2 | 25-01-2012 |
| | | | JP | 2008532062 A | 14-08-2008 |
| | | | US | 2008218589 A1 | 11-09-2008 |
| | | | WO | 2006087663 A1 | 24-08-2006 |
| US 2007008404 | A1 | 11-01-2007 | AU | 2003234904 A1 | 26-11-2004 |
| | | | US | 2007008404 A1 | 11-01-2007 |
| | | | WO | 2004100564 A1 | 18-11-2004 |
| US 2009268010 | A1 | 29-10-2009 | CN | 102076259 A | 25-05-2011 |
| | | | CN | 102188222 A | 21-09-2011 |
| | | | CN | 102188229 A | 21-09-2011 |
| | | | CN | 102197990 A | 28-09-2011 |
| | | | CN | 102197991 A | 28-09-2011 |
| | | | EP | 2303096 A1 | 06-04-2011 |
| | | | EP | 2641529 A1 | 25-09-2013 |
| | | | EP | 2641530 A1 | 25-09-2013 |
| | | | EP | 2641531 A1 | 25-09-2013 |
| | | | KR | 20110018330 A | 23-02-2011 |
| | | | US | 2009268010 A1 | 29-10-2009 |
| | | | US | 2009268011 A1 | 29-10-2009 |
| | | | US | 2009268012 A1 | 29-10-2009 |
| | | | US | 2009268015 A1 | 29-10-2009 |
| | | | US | 2009270678 A1 | 29-10-2009 |
| | | | WO | 2009131840 A1 | 29-10-2009 |
| US 2012062560 | A1 | 15-03-2012 | US | 2012062560 A1 | 15-03-2012 |
| | | | WO | 2012034113 A2 | 15-03-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012223446 A